# EUROPEAN PATENT APPLICATION

(11) **EP 3 939 580 A1**
(43) Date of publication of application: **19.01.2022**
(21) Application number: 21195048.0
(22) Date of filing: 30.09.2015
(51) Int. Cl.: A61K 31/202, A61K 31/593, A61K 31/702, A61K 31/7072, A61P 1/10

(54) **NEW COMPOSITION FOR PREVENTING GASTROINTESTINAL DISORDERS**

(30) Priority: 30.09.2014 WO PCT/NL2014/050672
(62) Divisional of application: 15818076.0
(71) Applicant: N.V. Nutricia, 2712 HM Zoetermeer (NL)
(72) Inventor: ATTALI, Amos, 2712 HM Zoetermeer (NL)
(74) Representative: V.O.

(57) **Abstract**

The present invention provides for a composition comprising a uridine source and butyrate producing fibers for use in the prevention or treatment of constipation, transit time dysfunction or colon length disorder. Said composition may also be used for the improvement of stool consistency. Advantageously said composition further comprises omega-3 polyunsaturated fatty acids such as DHA and/or EPA and a vitamin supplement comprising at least vitamin D3.

## Description

The invention relates to a new composition, preferably a food or feed composition comprising a uridine derivative and dietary fibers, preferably butyrate producing fibers, for the prevention or treatment of gastrointestinal disorders. The term GI-disorders covers a wide group of distinct disorders, varying from appendicitis, ileus, colitis, Crohn's disease, peptic ulcer, to anal fistulas, including enhanced or impaired motility of the gastro-intestinal tract (GIT) (too fast in diarrhea, too slow in slow-transit constipation), and changes in the contractility of the rectal sphincter muscles (leading to fecal incontinence or fecal impaction /obstipation). In the present invention particularly the composition is active against constipation, transit time dysfunction, colon length disorder and can be used for improving stool consistency.

Constipation is a common cause of painful defecation. Severe constipation includes obstipation (failure to pass stools or gas) and faecal impaction, which can progress to bowel obstruction and become life-threatening.

Constipation is a symptom with many causes. These causes are of two types: obstructed defecation and slow transit (or hypomobility) in at least a part of the gastrointestinal tract. Known causes of slow transit constipation include diet, hormonal disorders such as hypothyroidism, side effects of medications, low-grade inflammation (also referred to as chronic inflammation), food allergies, and rarely heavy metal toxicity. Treatments include changes in dietary habits, laxatives, enemas, biofeedback, and in particular situations surgery may be required.

In a review by Leung et α/. (JABFM July-August 2011, Vol 24(4): 436-450), it is observed that different forms of constipation (notably constipation either caused by slow colonic transit or pelvic floor dysfunction) require different forms of treatment.

EP-A-2 305 241 mentions that a food composition comprising 5'-nuclotide(s) in food which may be mixed with a binder/weighting agent such as starch may be used for treatment or prevention of upper gastrointestinal symptoms, such as dysphagia, gastroesophageal reflux disease, heartburn and vomiting. Although they mention 5'-uridylic acid as possible active ingredient, they only show examples with monosodium glutamate or sodium guanylate.

In WO 2007/061810 it has been described that amongst a large number of other compounds uracil may be given as a sweet taste improving additive to a sweetener composition containing further a high potency sweet compound (preferably rebaudioside) with a dietary fiber as active ingredient. Prevention of constipation is one of the effects that is mentioned when using dietary fibers as active ingredients. Nowhere in the document a relation between uridine and gastrointestinal conditions has been described.

US 2013/0189398 relates to an infant formula, which may comprise corn starch (as thickening agent), lower chain polyunsaturated fatty acids (LC PUFAs), and may be enriched with a mixture of nucleotides comprising UMP (1 mg to 15 mg per 100 g powdered formula) and/or non-digestible oligosaccharides such as fructo-oligosaccharides (FOS) and glucoo-oligosaccharides (GOS). The formula is especially suitable for treating/preventing esophageal reflux, colic and/or constipation. For UMP no specific function with relation to the gastrointestinal conditions has been described.

WO 2013/068879 is related to an infant formula comprising oligofructose and sn-2 palmitic acid. To this formula carbohydrates (such as maltodextrins, etc.), LC-PUFAs (such as DHA) and also nucleotides (such as UMP) may be added. A diet with the formula would lead to a reduction of fecal palmitic acid soaps, which would mean reduced constipation and an improved gastrointestinal tolerance. Also here no effect of uridine on the gastrointestinal condition has been stipulated.

Lastly, WO 2008/153377 relates to infant nutrition comprising two different non-digestible oligosaccharides, of which one may be FOS and an amount of non-viable Bifidobacterium. This composition may further be suppleted with 3-n PUFA (DHA) and it could contain other minerals, trace elements and vitamins and other essential components of which one may be UMP. The composition has been found to reduce stool problems in infants (both constipation and diarrhea). Also bowel diseases (irritable bowel syndrome, inflammatory bowel disease) have been mentioned.

However, none of the above prior art documents discloses that compositions (f)or foods comprising a uridine derivative and butyrate producing fibers may provide beneficial effects on constipation, transit time dysfunction, colon length disorder and/or that such a composition or food may be used for improving stool consistency.

It is an object of the present invention to provide a combination of specific components for use in the treatment of constipation, in particular for use in the treatment of slow transit constipation.

The terminology, slow transit constipation, was first coined in 1986 in a group of women who all displayed slow total gut transit time with a normal caliber colon in addition to a variety of other systemic symptoms (Preston Gut 1986).

It has now been found that a composition according to the invention is effective in the treatment of constipation, in particular in that transit time through the gastro-intestinal tract is improved. The inventors found that a composition comprising a uridine source and butyrate producing fibers may enhance transit through the whole length of the digestive tract (from stomach to rectum), greatly alleviating constipation.

In addition it was found that this composition can significantly reduce formation of protein alpha-synuclein aggregates in the gastro-intestional tract (GIT), which e.g. can be detected in humans by biopsy of the sigmoid colon, possibly acting via TLR4.

TLR4, meant for Toll-like receptor 4, is a receptor binding to LPS (lipopolysaccharides) from GRAM negative bacteria, also present on neuronal membranes, and is involved in the transport of alpha-synuclein into the cells. Although not shown herein, there is experimental evidence that TLR4 knock-out mice subjected to rotenone treatment (an animal model of constipation, see examples) fail to show alpha-synuclein depositions and have a normal transit time, strongly indicating that TLR4 is required for the endocytosis of alpha-synuclein into the neurons of the myenteric plexus, and that this deposition accounts for the impairments in transit time.

The inventors found that a composition comprising a uridine source and butyrate producing fibers was effective in reducing the transit time of stool through the intestinal system. In particular, the inventors found that such a composition is useful in the treatment of slow transit constipation. This is evidenced by the Examples, in which a mouse model was used.

The invention is in particular considered useful for treatment of a human, preferably a human of at least 18 years of age, in particular a human of at least 50 years of age, more in particular an elderly human (at least 65 years of age).
Figure 1 shows Immuno-histological staining of an antibody (brown color) that detects alpha-synuclein in the colons of the experimental groups of mice (Example 1).

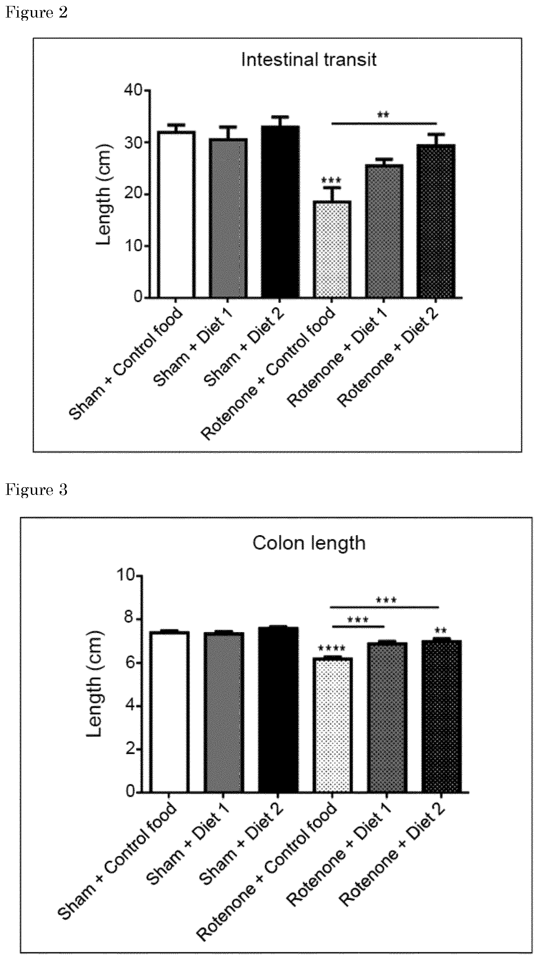

Figure 2 shows an effect of a diet with the composition of the present invention (diet 2) on intestinal transit.
Figure 3 shown an effect of a diet with the composition of the present invention (diet 2) on colon length.

The term "a" or "an" as used herein is defined as "at least one" unless specified otherwise.

When referring to a noun (*e.g.* a compound, an additive *etc.*) in the singular, the plural is meant to be included.

The term "or" as used herein is to be understood as "and/or" unless specified otherwise.

The term "constipation" refers in particular to its most used definition, i.e. for a human, evacuation of feces that is less than normal, preferably less than three times a week.

The term 'stool consistency' in the present application is used for the form and appearance of the stool (as e.g. indicated by the Bristol Scale), which is an indication of intestinal malfunctioning. In constipation the score of the stool on the Bristol Scale is 1 or 2, while normal stool would score 3-4 (and diarrhea scores 5-7).

The most widely used and accepted method of investigating constipated patients is the radio-opaque (solid markers) method (see for example Preston and Lennard-Jones, Gut, 1986, 27, 41-48).

The term 'fatty acid' is used herein in a way as is common in the prior art. Thus, fatty acid may include a free fatty acid or salt thereof or in a derivative form - suitable for use in a nutritional of pharmaceutical product - which derivative form is degradable in the body to release the fatty acid. Suitable derivative forms include esters and ethers, including monoglyceride, diglyceride, triglyceride and phospholipid forms, as known in the art. In particular, good results have been achieved with a triglyceride.

When making calculations about amounts, one can assume the same bioavailability as the pure fatty acid and a contribution of fatty acid that is similar to the amount moles of fatty acids in the complete molecule, and correcting for the weight of the complete molecule.

When referred to a dosage of a component, the specified dosage is in particular useful for treatment of a human, more in particular for an adult human, unless indicated otherwise.

The combination for use according to the invention is usually administered at least about once a week. Preferably it is at administered at least once per period of three days, more preferably at least once per period of two days, in particular at least once per day, more in particular at least twice per day. In general, the combination is administered up to 10 times, preferably up to 8 times, in particular 5 times per day or less. For embodiments wherein the combination or composition is administered less than once a day, the unit dosages (dosage per serving) of the active ingredients is usually within the range for the daily dosages mentioned elsewhere herein, although the concentration of the active ingredients may be higher.

A composition for use according to any of the invention can be administered as part of a nutritional composition. The nutritional composition may be a fluid drinkable composition, a spoonable composition or a solid composition. Preferably, the nutritional composition is a liquid composition, preferably liquid ready to drink composition. The composition or nutritional composition according to the invention is preferably administered to the subject in need thereof by oral ingestion. In an alternative embodiment, tube feeding is used.

In particular, in case a nutritional composition for use in accordance with the invention is intended for prophylactic or therapeutic treatment of symptom(s) of a disease, the composition may be a medical food product, *i.e.* a food product for use in enhancing, maintaining or restoring health and/or prevent a disease, prescribed by a health care professional like a physician, nurse, or dietician, and destined for and supplied to persons in need thereof.

In yet another embodiment, the combination is administered rectally.

The uridine source typically is selected from uracil, (deoxy)uridine, nucleotides of (deoxy)urdine, cytosine, (deoxy)cytidine, nucleotides of (deoxy)cytidine and derivatives thereof. Dietary cytidine, which is capable of being converted into uridine in humans, is regarded as a source of uridine in the context of the present invention.

Regarding the derivatives, in particular an amino group attached to the heterocyclic six membered ring may be derivatised or one or more of the hydroxyl groups of the ribose or deoxyribose of the nucleoside or nucleotide may derivatised, preferably esterified (acylated), for instance with a C1-C24 carboxylic acid. Preferred acylated forms of the uridine source are those wherein the (deoxy)ribose has been acylated with acetic acid, n-caproic acid, caprylic acid, or n-capric acid, because these increase the bioavailability of the uridine source. Methods for reacting these medium chain fatty acids to uridines, for example to the 5' position of the uridine are known in the art per se for other fatty acids and comprise conventional acylation methods. In a further embodiment the uridine source is acylated with a PUFA, for instance an omega-3 PUFA. Thus, in a specific embodiment, the combination for use of the invention comprises the PUFA and the uridine source combined in a single molecule, which may be hydrolysed in vivo. Esters of a uridine/cytidine derivatives and methods for making those are generally known in the art. Such derivatives have amongst others been described in EP 1,390,378 and in US 5,470,838.

Preferred uridine sources in a combination for use according to the invention the uridine source are selected from the group of uracyl, uridine, deoxyuridine, derivatised uridine, derivatised deoxy uridine, cytosine, (deoxy)cytidine and derivatised cytidines; more preferably a uridine source is selected from the group of UMP, UDP, UTP, CMP, CDP, CTP dUMP, dUDP, dUTP, dCMP, dCDP and dCTP. Optionally one or more hydroxyl moieties of the (deoxy)ribose of said nucleotide or nucleoside is acylated.

Particularly preferred is a uridine source selected from the group of UMP, UDP, UTP, dUMP, dUDP, dUTP,wherein optionally the (deoxy)ribose of said nucleotide or nucleoside is acylated.

In a highly preferred embodiment, the present combination for use according to the invention comprises uridine and/or uridine phosphate, more preferably it comprises one or more uridine phosphates selected from uridine monophosphate (UMP), uridine diphosphate (UDP) and uridine triphosphate (UTP), and more preferably UMP.

Preferably at least 50 wt.% of the uridine source in the combination for use according to the invention is provided by UMP, more preferably at least 75 wt.%, most preferably at least 95 wt.%.

The present combination for use according to the invention is preferably administered (or is in a format) to provide uridine (the cumulative amount of uridine, deoxyuridine, uridine phosphates, uracil and acylated uridine derivatives) in an amount of 0.08-3 g per day, preferably 0.1-2 g per day, more preferably 0.2-1 g per day.

The present combination for use according to the invention preferably comprises uridine in an amount of 0.08-3 g UMP per 100 ml of a liquid product, preferably 0.1-2 g UMP per 100 ml of a liquid product, more preferably 0.2-1 g per 100 ml liquid product.

Preferably 1-37.5 mg UMP per kilogram body weight is administered per day.

The required dosages of the equivalents on a weight base can be calculated from the dose for UMP by taking equimolar amounts using the molecular weight of the equivalent and of UMP, the latter being (about) 324 Dalton. Accordingly a daily dosage of the uridine source in the range of 3-116 µmol per kg body weight per day is preferred. If desired a lower amount, e.g. from about 1 to about 150 µmol/kg body weight can be administered.

The fibres may be selected from soluble fibres and insoluble fibres.

The fibres are generally composed of a plurality of carbohydrate units. The fibres may be short-chain indigestible carbohydrates or long-chain indigestible carbohydrates. Dependent on the type of fibre, national food regulations may have different definitions for what constitute short chain or long chain. As used herein, short chain fibres generally have a polymerization degree of less than 20, in particular of 2-12 less, more in particular 3-9; long chain fibres have a polymerization degree at least one higher than specified for short chain fibres, so 10 or more, 13 or more or 20 or more.

The inventors surprisingly found that the presence of a specific group of fibres in combination with the omega-3-PUFA and the uridine source has a positive effect on constipation. As illustrated by the Examples, mice treated with a diet containing butyrate producing fibre had positive effects on colon length and intestinal transit time.

Butyrate producing fibres are indigestible carbohydrates that have butyrate as a breakdown product when fermented by colonic flora. The term 'butyrate producing fibre' is used herein in particular for fibre that is capable of producing at least 0.5 mmol or more, preferably at least 0.75 mmol or more, more preferably at least 1.0 mmol or more butyrate / gram fibre after 24 hours of *in vitro* fermentation In practice, the maximum amount of butyrate producible under these condititions is usually 5 mmol or less, in particular 3.5 mmol or less, more in particular 2.5 mmol or less butyrate / gram fibre after 24 hours of *in vitro* fermentation.

The producible amount of butyrate is determinable using a semi-dynamic colon model (e.g. using a TIM artificial gut system). In the alternative, fresh faecal samples are collected from healthy adults (without gastrointestinal problems; no use of antibiotics for a last 2 weeks prior to sample taking). Faecal samples are divided in smaller portions and mixed with glycerol (10%) in an anaerobic cabinet and stored at -80°C. In each experiment the faecal samples from all donors are pooled at equal concentrations and mixed together in an anaerobic cabinet, to avoid subject-dependent variation in the adult microbiota as much as possible. The samples are inoculated with a fibre under fermentation conditions and fermentation is allowed to take place. Butyrate production is determined after 24 hrs using GC. In particular
- the model makes use of samples from four healthy adults (e.g., three male donors and one female donor) in the age of 19-35 years;
- The fibre is inoculated in a faeces suspension at a content of 200 mg/ 6 ml faeces suspension;
- The faeces suspension is made by mixing aeces with a fermentation medium as 1:5 v/v;
- The fermentation medium: buffered peptone water 3.0 g/1, Yeast Extract 2.5 g/1, Tryptone 3.0 g/1, L-Cysteine-HCl 0.4 g/1, Bile salts 0.05 g/1, K2HPO4.3H2O 2.6 g/1, NaHCO3 0.2 g/1, NaCl 4.5 g/l, MgSO4.7H2O 0.5 g/1, CaCl2. 2H2O 0.3 g/1, FeSO4.7H2O 0.005 g/1. Ingredients can be added one by one in 800 ml water, pH is adjusted to 6.3±0.1 with K2HPO4 or NaHCO3 and volume is filled up to 1 liter. Medium is sterilized for 15 minutes at 121°C;
- The fermentation temperature is 37 °C.

The total dosage of butyrate producing fibre in accordance with the invention preferably is 1 to 15 g per dosage, more preferably of 2 to 10 g per dosage, in particular 3-8 g per dosage. The total butyrate producing fibre content, based on total fibre content in a composition for use in accordance with the invention is up to 100 wt %, in particular 99 wt. % or less, more in particular 95 wt. % or less. The total butyrate producing fibre content, based on total fibre content preferably is at least 50 wt. %, based on total fibre content, preferably at least 70 wt. %, more preferably at least 80 wt. %, in particular 90 wt. % or more.

Preferably the combination or nutritional composition for use according to the invention comprises fibre in an amount sufficient to produce 0.3-5 mmol, more preferably 0.5-3.5, in particular 0.7-3, more in particular 1.0-2.5 mmol butyrate per gram fibre, under the using a semi-dynamic colon model, described above.

Preferred soluble butyrate producing fibres include fructooligosaccharides (FOS), galactooligosaccharides (GOS), bran and dextrins (e.g. Nutriose^{®}). These are well soluble and are a suitable substrate for colonic flora to produce butyrate, when fermented. Preferred brans are oat bran, rice bran and wheat bran. In a particularly preferred embodiment, several butyrate producing fibres are used, such as at FOS and a dextrin plus optionally bran and/or GOS; or FOS and bran plus optionally dextrin and/or GOS. Good results have been achieved with a combination of short chain FOS, long chain FOS, oat bran, GOS and dextrin. In an other preferred embodiment, the combination is part of a nutritional composition that comprises one or more butyrate producing fibres but that is essentially free of GOS. Such composition preferably is a milk-free composition.

Preferred insoluble butyrate producing fibres include resistant starch, such as high-amylose starch or retrograded or RS3 starch. Resistant starch is suitable to provide a particularly high butyrate production per g of resistant starch.

Resistant starch is defined to be as those starches which remain intact after digestion during 2 hours in the system of Englyst et al Am J Clin Nutr 1999, 69, 448-454. Commercially available resistant starches are Actistar and Novelose 330.

Preferred resistant starches are resistant starches from rice or corn. In a specific embodiment, the resistant starch comprises more than 50wt% linear polymers of alpha 1,4 glucans which have a degree of polymerization between 10 and 35. Suitable sources of such resistant starches are beans, peas, heat-treated potatoes and heat-treated cereals. Simultaneous presence in the colon of resistant starch and beta glucans, in combination with a xylan will support of growth of the right type of butyrate generating bacteria species.

In specific embodiment, the fiber comprise bran. Oat bran is an example of a good source for a dietary fiber for use in accordance with the invention.

The composition is preferably administered at least once per day, in particular 2-10 times per day. However, in certain circumstances, the composition may also be served less than once a day. In such a scheme the serving may comprise higher concentrations of active ingredients (uridine, butyrate producing fiber, vitamin D3 and polyunsaturated fatty acids).

In a preferred embodiment the composition also comprises omega-3 polyunsaturated fatty acids, preferably provided as fish oil. More preferably, the omega-3 polyunsaturated fatty acids are selected from the group consisting of docosahexaenoic acid (DHA), docosapentaenoic acid (DPA), and eicosapentaenoic acid (EPA).

Preferably, the daily dosage of DHA administered via the combination is in the range of 300 mg to 4000 mg, more preferably in the range of 500 mg to 2500 mg.

Preferably, the daily dosage of DHA plus EPA administered via the combination is in the range of 400 mg to 5000 mg, more preferably in the range of 500 mg to 3000 mg, in particular in the range of 1000 mg to 2500 mg.

Preferably, a composition for use according to the invention comprises fish oil providing polyunsaturated fatty acid having 18-24 carbon atoms.

Preferably, the combination for use according to the invention comprises at least one omega 3-PUFA, more preferably at least one PUFA selected from the group of docosahexaenoic acid (DHA), docosapentaenoic acid (DPA) and eicosapentaenoic acid (EPA).

The DHA, EPA and/or DPA are preferably provided as triglycerides, diglycerides, monoglycerides, free fatty acids or their salts or esters, phospholipids, lysophospholipids, glycerol ethers, lipoproteins, ceramides, glycolipids or combinations thereof.

More preferably, the composition for use according to the invention comprises at least DHA in triglyceride form.

Preferably, the composition for use according to the invention comprises DHA and EPA in sufficient amount for the administration of 400 to 5000 mg (DHA+EPA) per day, more preferably 500 to 3000 mg per day, most preferably 1000 to 2500 mg per day.

The proportion of (DHA+EPA) of the total fatty acids present in the combination is preferably 5-50 wt.%, more preferably 10-45 wt.%, most preferably 15-40 wt.%.

More preferably, the present composition for use according to the invention comprises DHA in sufficient amount for the administration of DHA in an amount of 300 to 4000 mg per day, more preferably 500-2500 mg per day.

The present composition for use according to the invention preferably contains a very low amount of arachidonic acid (AA).

The present composition for use according to the invention preferably comprises less than 5 wt.% arachidonic acid based on total fatty acids, more preferably below 2.5 wt.%, e.g. down to 0.5 wt%.

Preferably the weight ratio DHA/AA in the present composition for use according to the invention is at least 5, preferably at least 10, more preferably at least 15, preferably up to e.g. 30 or even up to 60.

The ratio omega-6/omega-3 fatty acids in the present combination for use according to the invention is preferably below 0.5, more preferably below 0.2, e.g. down to 0.05 or to 0.01. In particular, the ratio omega-6/omega-3 fatty acids (C 20 and higher) in the present combination for use according to the invention is preferably below 0.3, more preferably below 0.15, e.g. down to 0.06 or to 0.03.

The present composition for use according to the invention preferably comprises 1-40 wt.% DHA based on total fatty acids, preferably 3-36 wt.% DHA based on total fatty acids, more preferably 10-30 wt.% DHA based on total fatty acids.

The present composition for use according to the invention preferably comprises 0.5-20 wt.% EPA based on total fatty acids, preferably 2-10 wt.% EPA based on total fatty acids, more preferably 5-10wt.% EPA based on total fatty acids.

The daily dosage of polyunsaturated fatty acid having 18-24 carbon atoms administered via the combination usually is in the range of 0.4-15 grams, preferably in the range of 0.6-10 g, in particular in the range of 1-5 g.

In particular, good results have been achieved with a combination comprising both EPA and DHA. If both are present, the weight to weight ratio EPA:DHA usually is in the range of 1:99 to 99:1, preferably in the range of 1:10 — 5:1, in particular in the range of 1:5 — 1:2.

If EPA and/or DHA are present, the total daily dosage of DHA plus EPA taken together preferably is in in the range of 300-5000 mg/day, preferably in the range of 500 - 3000 mg/day, in particular in the range of 1000-2500 mg/day.

In an advantageous embodiment, fish oil or algae oil is suitable as a source of PUFA. A fish oil is particularly suitable as a source for the PUFA.

In a specific embodiment, the nutritional composition comprises one or more free amino acids or salts thereof.

In a highly preferred embodiment, the combination for use in accordance with the invention comprises vitamin D. Good results have been obtained with vitamin D3 (cholecalciferol, calcifediol, calcitriol).

If present, the concentration of vitamin D, preferably of vitamin D3, in a nutritional composition for use in accordance with the invention usually is in the range of 5-110 µg/100, in particular in the range of 6-85 µg/100g preferably in the range of 10-50µg/100g, more preferably 15-45 µg/100g.

The combination for use according to the invention usually provides vitamin D to the mammal, in particular human, that is treated in a in a daily dosage of up to about 50 µg, preferably 25 -40 µg. For a liquid product, the vitamin D3 content preferably is 5-85 µg per unit dosage. The unit dosage of a liquid product preferably has a volume of 50-250 ml, in particular 100-150 ml.

In a specific embodiment, the nutritional composition for use according to the invention comprises one or more trace elements, in particular selenium.

In a specific embodiment, the nutritional composition comprises one or more trace elements, preferably selenium.

Preferably a composition of the invention, in particular a nutritional composition to be used according to the invention further comprises a vitamin, preferably at least one vitamin selected from the group of, vitamin E, vitamin C, vitamin D, vitamin B12, vitamin B6 and folic acid;

Advantageously, vitamin B12 and folate are included.

A nutritional composition for use in accordance with the invention preferably comprises 50 to 1000 µg folic acid, more preferably 150 to 750 µg, most preferably 200 to 500 µg folic acid, per 100 ml liquid product.

The daily folic acid dosage in accordance with the invention preferably is 50-1000 µg folic acid per day, more preferably 150-750 µg, most preferably 200 - 500 µg folic acid per day.

The present nutritional composition preferably comprises 0.5 to 15 µg vitamin B12, more preferably 1 to 10 µg, most preferably 1.5 to 5 µg vitamin B12, per 100 ml liquid product.

The daily vitamin B 12 dosage in accordance with the invention preferably is 0.5-15 µg vitamin B12 per day, more preferably 1-10 µg, most preferably 1.5-5 µg vitamin B12 per day.

Advantageously, the nutritional composition in a use according to the invention comprises 0.5 to 3 mg, preferably 0.5-2 mg vitamin B6, per 100 ml liquid product.

Preferably, each of vitamin B6, vitamin B12 and folate are included.

Preferably, the nutritional composition comprises at least 2, 3, 4, 5, or 6 components selected from the group o of phospholipids, vitamin E, vitamin C, selenium, vitamin B12, vitamin B6 and folic acid, even more preferably comprises phospholipids, vitamin E, vitamin C, selenium, vitamin B12, vitamin B6 and folic acid.

In a specific embodiment, the composition contains choline. Preferably the present nutritional composition contains choline and/or phosphatidylcholine. Preferably the nutritional composition is administered (or is in a format) for providing more than 50 mg choline per day, preferably 80-2000 mg choline per day, more preferably 120-1000 mg choline per day, most preferably 150-600 mg choline per day.

Preferably the present nutritional composition contains 50 mg to 3 gram choline per 100 ml of a liquid formula, preferably 200 mg - 1000 mg choline/100 ml.

The present nutritional composition may advantageously contain phospholipids, preferably provided in the form of lecithin. The present nutritional composition is preferably a liquid composition, wherein lecithin is provided in an amount of 0.01 and 1 gram lecithin per 100 ml, more preferably between 0.05 and 0.5 gram lecithin per 100 ml.

The nutritional composition usually further comprises a digestible carbohydrate fraction and/or a proteinaceous matter fraction (peptides, proteins, amino acids, including derivatives thereof that still contribute to energetic value of the composition, e.g. hydrolysable esters). Further, the composition may comprise a lipid fraction comprising the PUFA and one or more other lipids.

In an advantageous embodiment, the lipids account for less than 50 en% of the nutritional composition, in particular 46 wt. % or less, more in particular 43 wt. % or less, or 40 wt. % or less. Usually, the lipids (including he omega-3 PUFA) provide at least 1 en %, preferably at least 5 en %, in particular at least 10 en % of the nutritional composition. Proteinaceous matter and/or digestible carbohydrates (non-lipid component having a caloric value ) typically provide the balance of the energetic value. For energetic value the Atwater factors are used: for digestible carbohydrates (4 kcal/g), proteins (4 kcal/g) and lipids (9 kcal/g), and zero for others, such as the organic acids and nutritional fiber, minerals and vitamins.

The nutritional composition may be a fluid drinkable composition, a spoonable composition or a solid composition. Preferably, the nutritional composition is a liquid composition, preferably liquid ready to drink composition.

The (nutritional) composition according to the invention, is preferably administered to the subject in need thereof by oral ingestion. In an alternative embodiment, tube feeding is used.

In yet another embodiment, the combination is administered rectally.

A combination for use according to the invention, or a nutritional composition of the invention, including any embodiment described therein, is preferably for preventing or treating constipation, in particular slow transit constipation (also referred to as slowed transit constipation), in a subject suffering from, or at risk of suffering from a neurological disorder.

It is acknowledged as a general experience that constipation is frequent in acute admitted stroke patients. One possible reason could be an impaired coordination of the peristaltic wave and the relaxation of the pelvic floor and external sphincter.

Gastrointestinal dysfunctions is frequent and sometimes a dominant symptom of Parkinson's disease (PD), where constipation could result from a decreased bowel movement frequency. It has been reported that more than 50% of PD patients suffer from moderate to severe constipation. Some of these PD patients had pathologically prolonged colon transit time, in other words suffer from slow transit constipation. The reduction of the alpha-synuclein aggregates in the colon might favour a better functioning of the autonomic nervous system (ANS) and/or the enteric nervous system (ENS), and therefore allow a better functioning of the peristalsis of the gastrointestinal tract, and more generally a better functioning of the digestive process (meant to encompass ingestion, digestion, absorption of the nutrients and defecation).

Moreover, it has been shown that anticholinergic agents, and Levo-dopa (1-dopa), by slowing down the bowel movements, enhance the constipation problem.

It has been shown that slow-transit and gastroparesis causes the degradation of 1-dopa into dopamine in the intestinal tract. Dopamine in turn inhibits cholinergic fibers and execerbates respectively the problems of slow transit and delayed gastric emptying even further.

Studies have shown that the 1-dopa is more effective when the constipation problem is addressed simultaneously.

Therefore, a composition for use according to the invention, or a nutritional composition of the invention can be used in parallel (i.e. administered simultaneously, or before, or after) to the use of an anticholinergic agent for treating PD, such as benzhexol, orphenadrine, benztropine, bornaprine, benapryzine, or mithixine, or to the use of 1-dopa.

A composition for use according to the invention, or a nutritional composition of the invention can be used for treating synucleopathies related/associated constipation, including (besides PD) dementia with Lewy bodies, and multiple system atrophy.

Bowel dysfunctions in patients with multiple sclerosis is common. Possibly through the attenuation of chronic (low grade) inflammation, attenuation possibly obtained by down regulating TLR4, a combination for use according to the invention, or a nutritional composition of the invention is expected to improve the constipation issues, in particular the slow transit constipation.

It is well documented that patients having spinal cord injury suffer from prolonged colonic transit time, resulting in slow transit constipation. Possibly through the better functioning of the autonomic nervous system (ANS) and/or the enteric nervous system (ENS), a combination for use according to the invention, or a nutritional composition of the invention is expected to improve the constipation issues, in particular the slow transit constipation.

There are other diseases affecting the peripheral nervous system and/or motor neurons to which constipation is associated. Examples of such diseases are diabetic neuropathy, pure autonomic failure (PAF).

Possibly through the better functioning of the autonomic nervous system (ANS) and/or the enteric nervous system (ENS), a combination for use according to the invention, or a nutritional composition of the invention is expected to improve the constipation issues, in particular the slow transit constipation.
(See Winge et al., 2003, J Neurol Neurosurg Psychiatry; 74; 13-19)

Possibly through the attenuation of chronic (low grade) inflammation, attenuation possibly obtained by down regulating TLR4, a combination for use according to the invention, or a nutritional composition of the invention is expected to improve the constipation issues, in particular the slow transit constipation, in patients suffering from GI-disorders, such as ileus, colitis, inflammatory bowel disease (more particularly Crohn's disease or ulcerative colitis), and peptic ulcer.

The GI-disorders triggering chronic inflammation of the mucosa of the gastrointestinal tract enhance the disruption of the mucosal integrity, which activate TLR4.

Possibly through the attenuation of chronic (low grade) inflammation, attenuation possibly obtained by down regulating TLR4, a combination for use according to the invention, or a nutritional composition of the invention is expected to improve the constipation issues, in particular the slow transit constipation, in patients suffering from PDD, in particular ASD.

Since sometimes the normal medication that is give to treat or prevent the above mentioned neurological and/or psychiatric disorders, the invention also comprises the administration of the composition of the invent to counter the effects on obstipation caused by such medication. Accordingly, the invention comprises a method for the treatment of a neurological disorder and/or a psychiatric disorder, wherein the disorder is selected from the disorders listed above, wherein the treatment takes place by orally administering a composition according to the invention together with a medicament that is normally used for treatment of said neurological disorder and/or psychiatric disorder.

In summary, in particular the invention relates to the following items:
1. Composition comprising a uridine source and butyrate producing fibers for use in the prevention or treatment of constipation, transit time dysfunction or colon length disorder.
2. Composition comprising a uridine source and butyrate producing fibers for use in the improvement of stool consistency.
3. Composition for use according to item 1 or 2, further comprising at least one omega-3-polyunsaturated fatty acid, preferably wherein said fatty acid has 18 - 24 carbon atoms.
4. Composition for use according to item 3, where the omega-3-polyunsaturated fatty acid is chosen from the group consisting of docosahexaenoic acid (DHA), docosapentaenoic acid (DPA), and eicosapentaenoic acid (EPA).
5. Composition for use according to any of the previous items, wherein the uridine source comprises a nucleotide selected from the group of uridine, deoxyuridine , UMP, UDP, UTP, CMP, CDP, CTP dUMP, dUDP, dUTP, dCMP, dCDP and dCTP, wherein optionally one or more hydroxyl moieties of the (deoxy)ribose of said nucleotide are acylated.
6. Composition for use according to item 5, wherein the uridine source is selected from the group consisting of uridine, deoxyuridine, acylated uridine, acylated doxyuridine, non-acylated UMP and acylated UMP.
7. Composition for use according to item 5 or 6, wherein the daily dosage of the uridine source is in the range of 1-150 µmol per kg body weight per day, preferably in the range of 3-116 µmol per kg body weight per day.
8. Composition for use according to any of the previous items, wherein the butyrate producing fibre is selected from the group of fructooligosaccharides (FOS), galactooligosaccharides (GOS) and dextrins (e.g. Nutriose^{®}).
9. Composition for use according to any of the previous items, wherein the composition further comprises vitamin D, preferably vitamin D3.
10. Composition for use according to item 9, wherein vitamin D, preferably vitamin D3, is to be administered in a daily dosage of 5-50 µg, in particular 25-40 µg
11. Composition for use according to any of the previous items, wherein the composition is to be administered as a nutritional product or a part of a nutritional composition, further comprising at least one of phospholipids, vitamin E, vitamin C, selenium, vitamin B12, vitamin B6 and folic acid.
12. Composition for use according to any of the previous items, wherein the composition is to be administered into the gastrointestinal tract.
13. Composition for use according to any of the previous items, wherein the combination is part of a nutritional composition, further comprising at least non-lipid components having a caloric value selected from the group of proteinaceous matter and digestible carbohydrates, wherein the total caloric value of said non-lipid components is at least 50 en %, preferably 55 en% or more, in particular 60 en % or more.
14. Composition for use according to any of the previous items, wherein the composition is to be administered orally.
15. Composition for use according to any of the previous items, wherein the constipation is associated to a neurological disorder or a psychiatric disorder.
16. Composition for use according to item 15, wherein the neurological disorder is a peripheral neuropathy, an autonomic neuropathy, or an enteric nervous system neuropathy, preferably an autonomic neuropathy, or an enteric nervous system neuropathy, more preferably wherein the neurological disorder is selected to from the group consisting of synucleopathies, diabetic neuropathy, Duchenne's dystrophy, cerebrovascular disease, Multiple Sclerosis, pure autonomic failure, and spinal cord injury, preferably selected to from the group consisting of cerebrovascular disease, Multiple Sclerosis, pure autonomic failure, spinal cord injury, and Parkinson's disease.
17. Composition for use according to item 15, wherein the psychiatric disorder is pervasive development disorder, preferably autistic spectrum disorder, or the psychiatric disorder is depression, in particular a depressive mood disorder.
18. Method for the treatment of a neurological disorder and/or a psychiatric disorder, wherein the disorder is selected from the disorders listed in any of items 15 -17, wherein the treatment takes place by orally administering a composition as defined in any of items 1 — 12 together with a medicament that is normally used for treatment of said neurological disorder and/or psychiatric disorder.

The invention will now be illustrated by the following examples.

### Example 1

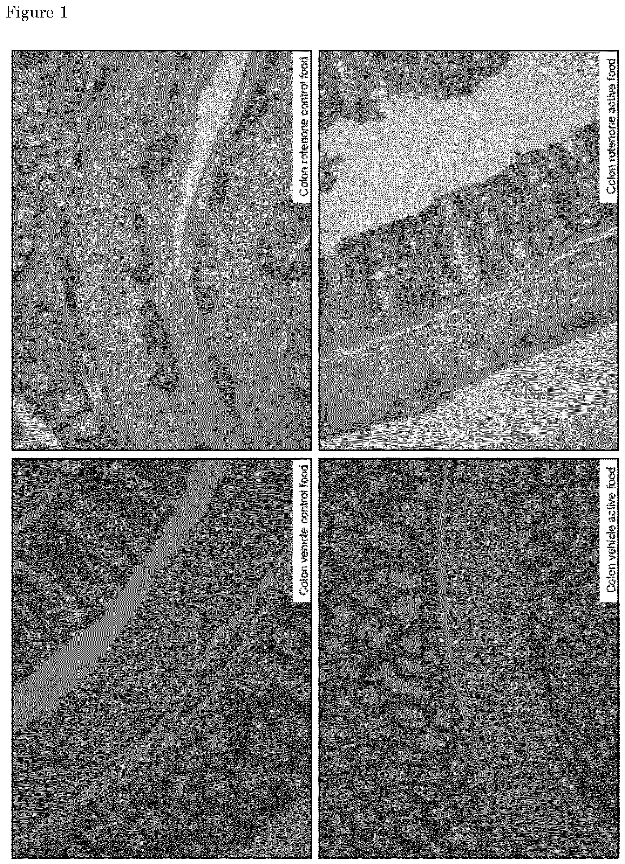

Constipation, the most important and debilitating non-motor symptom of Parkinson's disease (PD), was induced in mice by intra-striatal injection of 2.7 µg of rotenone by stereotaxic surgery into the brain. Epidemiological studies had previously shown that exposure to this pesticide induced Parkinson's disease (PD). In addition to the observed constipation, the intra-striatal injection with rotenone also resulted in the well-known motor symptoms of PD, shown by the impaired ability of the mice to remain on a moving rotarod (see Figure 1). The rotarod treadmill consists of a plastic rod, with a non-slippery surface, above the base (trip plate). Mice were placed on an accelerating rod with speeds starting with 2 rpm and gradually increasing to 20 rpm. The rodent's ability to remain on the rotating rod (time to first fall) was recorded for a maximum of 5 minutes. Before baseline, mice were trained with the rotarod apparatus to rule out learning effects throughout the experiment.

### Example 2

The effects of a diet comprising a pyrimidine derivative (UMP) and a PUFA source (DHA) in a therapeutic setting were studied, wherein treatment with a diet according to the invention was initiated 4 weeks after injection with 5.4 µg rotenone or a vehicle and continued for seven weeks. Six experimental groups of male C57B1/6J mice were used which were either treated with vehicle (Sham) or rotenone and which either received a control diet or a diet according to the invention (diet 1 or diet 2), as indicated in the following table:

**Table 1:. Experimental groups**

| Group | Intra-striatal injection | diet |
|---|---|---|
| Sham+Control Food (control) | Sham | Control |
| Sham+Diet 1 (control) | Sham | Uridine source+ DHA |
| Sham+Diet 2 (control) | Sham | Uridine source + DHA + butyrate producing fibres |
| Rotenone+Control Food | Rotenone | Control |
| Rotenone+Diet 1 | Rotenone | Uridine source + DHA |
| Rotenone+Diet 2 ) | Rotenone | Uridine source + DHA + butyrate producing fibres |

The compositions of the control and active diets are presented in the following Table.

**Table 2: Composition of the diets**

| animal food based on AIN -93 M Reeves, et al. (1993) | g / 100 g diet | g / 100 g diet | g/100 g diet |
|---|---|---|---|
| | Control | Diet 1: Fish oil+UMP | Diet 2: Fish oil+Uridine+but y-rate producing fibers |
| Corn starch | 31.10 | 29.30 | 31,97 |
| Casein (>85% protein) | 14.00 | 14.00 | 14,00 |
| corn dextrine | 15.50 | 15.50 | 14,20 |
| Sucrose | 10.00 | 10.00 | 10,00 |
| Dextrose | 10.00 | 10.00 | 10,00 |
| | | | |
| Fibers (cellulose) | 5.00 | 5.00 | 0 |
| Butyrate producing fibers | 0 | 0 | 7.27¹ |
| Minerals mix (AIN-93M-MX) (*) | 3.50 | 3.50 | 3.50 |
| Vitamins mix (AIN-93-VX) (*) | 1.00 | 1.00 | 1.00 |
| Fat | 4.77 | 4.77 | 4.77 |
| saturated fat | 1.3 | 1.2 | 1.2 |
| Monounsaturated fatty acids (MUFA) | 1.1 | 1.1 | 1.1 |
| PUFA | 2.3 | 2.2 | 2.2 |
| EPA (C-20:5w3) | 0.0 | 0.3 | 0.5 |
| DHA (C-22:6w3) | 0.0 | 0.7 | 0.7 |
| | | | |
| Additions: | | | |
| L-cystine | 0.180 | 0.180 | 0.180 |
| Choline bitartrate (41.1% choline) | 0.250 | 0.250 | 0.922 |
| Tert-butylhydroquinone | 0.0008 | 0.0008 | 0.0008 |
| Additional vitamins² | | | 0.688 |
| Soy lecithin | | | 0.755 |
| UMP disodium (24%H2O) | 0 | 1.0 | - |
| Uridine (pure) | | | 0.511 |
| Total | 100.0 | 100.0 | 100.0 |
| Energy | 344.6 | 345.5 | 358.0 |

| | | | |
|---|---|---|---|
| ¹ Butyrate producing fibres (5 gram per 100 g diet): 1.50 g GOS 0.17 g long-chain FOS 1.67 g short- chain FOS 1.67 g nutriose And further 0.5 g lactose and 0.5 g the carbohydrate and moisture to 7.27 g ² Additional vitamins (per 100 g diet) Ascorbic acid (100% pure) 0.16 g Vit. E (Tocopherol acetate, 50 %) 0.465 g Vitamine D (cholecalciferol, 500.000 IU/g) 5.6 µg Vit. B6 (Pyridoxin hydrochloride, 82 %) 0.00407 g Folic acid (100%) 0.00060 g Vit. B12 (Cyanocobalamin 0,1 %) 0.0575 g Na selenite • 5 H2O 0.00034 g | | | |

Diet 1 was composed of the control diet plus additionally UMP and DHA (fish oil).

Diet 2 was as Diet 1, without cellulose , but instead additionally a mixture of galactooligosaccharides and fructooligosaccharides (both long chain and short chain fructooligosaccharides and instead of UMP uridine.

When considering the effects on gastrointestinal functioning, it appeared that diet 2 increased intestinal transit and colon length, especially in mice treated with rotenone. However, also with sham treated rats increasing trends were observed.

## Claims

1. Composition comprising a uridine source, which uridine source is selected from the group of uridine, deoxyuridine , UMP, UDP, UTP, CMP, CDP, CTP, dUMP, dUDP, dUTP, dCMP, dCDP and dCTP, wherein optionally one or more hydroxyl moieties of the (deoxy)ribose of said uridine source are acylated, and butyrate producing fibers for use in the prevention or treatment of constipation, transit time dysfunction or colon length disorder.

2. Composition for use according to claim 1, wherein the composition is to be used in parallel to the use of 1-dopa.

3. Composition for use according to claim 2, wherein the composition is for use in the prevention or treatment of constipation and the 1-dopa to be used in parallel is more effective, compared to the use of 1-dopa without said use of said composition in parallel.

4. Composition for use according to any of the previous claims, wherein the composition is to be used parallel to the use of an anticholinergic agent for treating Parkinson's disease.

5. Composition for use according to claim 4, wherein the anticholinergic agent is selected from the group of benzhexol, orphenadrine, benztropine, bornaprine, benapryzine and mithixine.

6. Composition for use according to any of the previous claims, further comprising at least one omega-3-polyunsaturated fatty acid, preferably wherein said fatty acid has 18 - 24 carbon atoms, more preferably, wheren the omega-3-polyunsaturated fatty acid is chosen from the group consisting of docosahexaenoic acid (DHA), docosapentaenoic acid (DPA), and eicosapentaenoic acid (EPA).

7. Composition for use according to any of the previous claims, wherein the uridine source is selected from the group consisting of uridine, deoxyuridine, acylated uridine, acylated doxyuridine, non-acylated UMP and acylated UMP.

8. Composition for use according to any of the previous claims, wherein the daily dosage of the uridine source is in the range of 1-150 µmol per kg body weight per day, preferably in the range of 3-116 µmol per kg body weight per day.

9. Composition for use according to any of the previous claims, wherein the butyrate producing fibre is selected from the group of fructooligosaccharides (FOS), galactooligosaccharides (GOS) and dextrins (e.g. Nutriose^{®}).

10. Composition for use according to any of the previous claims, wherein the composition further comprises vitamin D, preferably vitamin D3.

11. Composition for use according to claim 7, wherein vitamin D, preferably vitamin D3, is to be administered in a daily dosage of 5-50 µg, in particular 25-40 µg.

12. Composition for use according to any of the previous claims, wherein the composition is to be administered as a nutritional product or a part of a nutritional composition, further comprising at least one of phospholipids, vitamin E, vitamin C, selenium, vitamin B12, vitamin B6 and folic acid.

13. Composition for use according to any of the previous claims, wherein the composition is to be administered into the gastrointestinal tract.

14. Composition for use according to any of the previous claims, wherein the combination is part of a nutritional composition, further comprising at least non-lipid components having a caloric value selected from the group of proteinaceous matter and digestible carbohydrates, wherein the total caloric value of said non-lipid components is at least 50 en %, preferably 55 en% or more, in particular 60 en % or more.

15. Composition for use according to any of the previous claims, wherein the composition is to be administered orally.

16. Composition for use according to any of the previous claims, wherein the constipation is associated to a neurological disorder or a psychiatric disorder, preferably to Parkinson's disease.
